(19) Europäisches Patentamt
European Patent Office
Office européen des brevets

(11) EP 3 124 554 A1

(12) **EUROPEAN PATENT APPLICATION**

(43) Date of publication:
**01.02.2017 Bulletin 2017/05**

(21) Application number: **15179176.1**

(22) Date of filing: **30.07.2015**

(51) Int Cl.:
***C09C 1/02*** *(2006.01)* ***C09D 7/12*** *(2006.01)*
***C08K 3/26*** *(2006.01)* ***C08K 7/08*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Imerys SA**
**75007 Paris (FR)**

(72) Inventor: **The designation of the inventor has not yet been filed**

(74) Representative: **Nash, David Allan et al**
**Haseltine Lake LLP**
**Redcliff Quay**
**120 Redcliff Street**
**Bristol BS1 6HU (GB)**

(54) **PRECIPITATED CALCIUM CARBONATE, A METHOD FOR ITS MANUFACTURE AND USES THEREOF**

(57) The present invention concerns precipitated calcium carbonate particles being at least partially in the form of nanofibers or nanochain like agglomerates, wherein the particles are at least partially coated with at least one coating agent, use of such precipitated calcium carbonate particles and a process for the manufacture of such precipitated calcium carbonate particles.

EP 3 124 554 A1

## Description

The present invention concerns precipitated calcium carbonate particles being at least partially in the form of nanofibers or nanochain like agglomerates, wherein the particles are at least partially coated with at least one coating agent, use of such precipitated calcium carbonate particles and a process for the manufacture of such precipitated calcium carbonate particles.

Precipitated calcium carbonate particles are used in a variety of materials, such as plastics and paints, often to replace TiO2 as pigment, generally to enforce the materials, modulate the characteristics of the materials, or to lower production cost. In some cases, it is desirable to provide precipitated calcium carbonate particles which exhibit certain opaque properties and/or high light scattering coefficient as additive in materials such as paints, papers and paper coatings, as described in WO2013/050495. Coated precipitated calcium carbonate particles can exhibit good properties when used as fillers in polymers and polymer compositions, for example in plastisols, enhancing the mechanical properties of the materials. Coating of precipitated calcium carbonate particles with coatings helps to increase the oleophilicity of the precipitated calcium carbonate particles, thus improving the dispersibility of the fillers in the polymers or polymer compositions, as described in WO2006/051087.

It has now been found that precipitated calcium carbonate particles being at least partially in the form of nanofibers or nanochain like agglomerates, wherein the particles are at least partially coated with at least one coating agent, exhibit surprisingly improved characteristics when used as additives in materials, in particular polymeric materials, specifically in plastisols. Exceptionally good rheological properties, such as high yield values, can be achieved by such particles in polymeric materials, in particular in plastisol compositions.

Preferred rheological properties in plastisols are for example a high viscosity at low shear such that the plastisol does not sag once applied. A low viscosity at high shear is desirable so that the plastisols can be applied by spraying. The rheological properties are suitable determined, for example, by their Yield Value, which should be sufficiently high. Yield Value can be defined as the stress at which a material begins to deform plastically. Further, good adhesion to a wide variety of substrates, for example concrete, steel, glass, fabrics or plastics, is aimed for. The additive must show a good dispersion of all particles to ensure homogeneity and equal properties, smooth, repeatable processes and to avoid clogging of application apparatus, such as spray nozzles. The precipitated calcium carbonate particles according to the present invention display significantly improved rheological properties, in particular in plastisols, excellent dispersion characteristics and polymeric compositions comprising the precipitated calcium carbonate particles have a very good stability, manufacturing and product properties.

Thus, the present invention concerns precipitated calcium carbonate particles being at least partially in the form of nanofibers or nanochain like agglomerates, wherein the particles are at least partially coated with at least one coating agent. The invention further relates to a process for the preparation of precipitated calcium carbonate particles being at least partially in the form of nanofibres or nanochain like agglomerates, wherein the particles are at least partially coated with at least one coating agent, which comprises a step of contacting precipitated calcium carbonate particles being at least partially in the form of nanofibres or nanochain like agglomerates with at least one coating agent. In a particular embodiment, the invention relates to a process for the preparation of precipitated calcium carbonate particles being at least partially in the form of nanofibers or nanochain like agglomerates, wherein the particles are at least partially coated with at least one coating agent, which comprises

(a) Precipitating calcium carbonate particles from a suspension comprising calcium hydroxide and water under conditions providing precipitated calcium carbonate particles being at least partially in the form of nanofibers or nanochain like agglomerates, and

(b) Contacting the particles of calcium carbonate obtained from step (a) with the at least one coating agent.

The invention further relates to the use of precipitated calcium carbonate particles according to the invention as additive, for example filler, in paints, plastisols, sealants, paper, food products, nutritional products, pharmaceutical products, inks, lacquers and polymers, in particular in plastisols. Paints, plastisols, sealants, papers, food products, nutritional products, pharmaceutical products, inks, lacquers and polymers and polymer compositions, in particular plastisols, comprising precipitated calcium carbonate particles according to the present invention are also subject of the invention.

It is understood that the citation of a singular also intends to include the plural in the present invention; for example, "coating agent" also intends to designate the plural "coating agents".

It is further understood that the word "comprises" in the present invention includes the term "consists of".

The precipitated calcium carbonate particles according to the present invention are at least partially in the form of nanofibers or nanochain like agglomerates. These can be described as constituted by at least two interconnected primary particles. These nanofibers may then aggregate themselves in a random or organized way, preferably to form microshells or microsheaves.

The term "particle" is understood to mean a physically and chemically autonomous entity. The term "primary particle" refers to the elementary particles of precipitated calcium carbonate.

In the terms "nanofibers" and "nanochain like agglomerate", the prefix "nano" means that the nanofibers or nanochain like agglomerates have a characteristic dimension at the nanoscale, in particular a characteristic dimension which is, on average, less than 100 nm. In nanofibers or nanochain like agglomerates, said characteristic dimension is the average diameter.

The term "nanofiber" intends to denote an elongated entity having a characteristic dimension, i.e. average diameter, less than 100 nm. The term "nanochain like agglomerate" intends to denote an elongated entity having a characteristic dimension, i.e. average diameter, less than 100 nm. Nanofibers mainly differ from nanochain like agglomerates in that the individual primary particles cannot be distinguished anymore and form nanofibers which appear to be homogeneous and even, for example on electron microscopy photographs, whatever the magnification. In nanochain like agglomerates, the primary particles retain their individuality and remain visible, for example on electron microscopy. Nanochain like agglomerates can also be named "nanorosaries". An essential feature of the present invention resides in the fact that at least part of the precipitated calcium carbonate particles are in the form of nanofibers or nanochain like agglomerates, such nanofibers or nanochain like agglomerates, which can be described as being constituted by at least two interconnected primary particles and therefore having an elongated morphology. In the present invention, the term "at least partially present in the form of nanofibers or nanochain like agglomerates" denotes that the precipitated calcium carbonate particles are generally present in the form of nanofibers or nanochain like agglomerates in an amount of at least 1 % by weight of the calcium carbonate particles. Often, precipitated calcium carbonate particles are present in the form of nanofibers or nanochain like agglomerates in an amount of at least 8 % by weight of the calcium carbonate particles. In the present invention, precipitated calcium carbonate particles are typically present in the form of nanofibers or nanochain like agglomerates in an amount of at least 10 % by weight of the calcium carbonate particles, more preferably in an amount of at least 15 % by weight of the calcium carbonate. The amount of nanofibers or nanochain like agglomerates has been evaluated relying on SEM (Scanning Electron Microscopy) or TEM (Transmission Electron Microscopy) image analysis. The obtained values correspond to the number of elementary particles that belongs to the nanofibers in respect to the total number of elementary nanoparticles, the measurement being performed in areas of acceptable resolution. It is preferred to determine the amount in a homogenized sample. For the determination using SEM, the calcium carbonate particles are metallized: The samples are directly placed on a graphite tape, then metallized with platinum for 1 minute under a vacuum of $10^{-1}$ Pa with a beam intensity of 6 mA. A Hitachi S-4800 SEM was used for the measurements.

According to the invention, the primary particles are preferably in the form of calcite crystals. The primary particles may be present in a huge variety of shapes, the most common being pseudo-spherical or cubic morphology. Scalenohedral primary particles can also be present when the primary particle is of the calcite type. In another aspect, the primary particles are in the form of aragonite crystals, which often display a needle-like morphology. Mixtures of the foregoing morphologies are also possible.

The nanofibers or nanochain particles of the precipitated calcium carbonate are, in one embodiment, further aggregated to form microshells or microsheaves. Microshells may be composed of tens to hundreds of nanofibers or nanochain like agglomerates. In such a case, the nanofibers or nanochain like agglomerates are usually visible at least on the inner part of the microshell like agglomerates. Microsheaves are also denoted as "faggots". The nanofibres or nanochains are aggregated parallel to one another, in a surprising organized fashion, in such a microsheave. Such a microsheave generally composed of several tens of similar nanofibres and/or nanochains. This number is preferably greater than 100. Microsheaves comprising more than 10 000 nanofibres and/or nanochains are exceptional.

In one aspect, the nanochains or nanofibres are aggregated in a random fashion.

According to one embodiment of the present invention, precipitated calcium carbonate particles which are at least partially present in the form of nanofibers or nanochain like agglomerates have an average specific surface area of equal to or lower than 60 $m^2g^{-1}$, preferably equal to or lower than 50 $m^2g^{-1}$ (measured by the BET (Brunauer-Emmett-Teller) technique described in ISO 9277). Generally, this value applies to the agglomerates before the coating step as well as after the coating step. In this embodiment, the specific surface of the calcium carbonate particles which are at least partially present in the form of nanofibers or nanochain like agglomerates before coating is preferably equal to or higher than 10 $m^2g^{-1}$, more preferably equal to or higher than 15 $m^2g^{-1}$, and most preferably equal to or higher than 20 $m^2g^{-1}$. Also, the specific surface of the calcium carbonate particles before coating is preferably equal to or lower than 60 $m^2g^{-1}$, more preferably equal to or lower than 50 $m^2g^{-1}$, and most preferably equal to or lower than 45 $m^2g^{-1}$. In particular, the specific surface of the calcium carbonate particles before coating is within the range of from 10 to 60 $m^2g^{-1}$, more preferably from 10 to 50 $m^2g^{-1}$, most preferably from 15 to 50 $m^2g^{-1}$ and in particular from 20 to 45 $m^2g^{-1}$. For the purpose of the specification, the specific surface of a precipitated calcium carbonate particle is defined as the surface area which is determined according to the BET-method (adsorption isotherm of nitrogen according to Brunauer-Emmett-Teller (BET)). According to the BET-method the surface of a powder can be calculated from the $N_2$-isotherm, which is observed at the boiling point of liquid nitrogen. For details, it can be referred to the ISO 9277 norm (1995-05-15).

The precipitated calcium particles according to the present invention are at least partially coated with at least

one coating agent. In one aspect, only one coating agent is present on the particles. According to the present invention, the at least one coating agent is selected from the group consisting of sulfur organic compounds, fatty acids and the salts thereof.

Generally, the at least one coating agent is comprised in an, if applicable combined, amount of from 0.01 wt% to 15 wt%. Generally, the amount of the at least one coating agent which is comprised in the particles according to the present invention, is equal to or more than 0.01 wt%, preferably equal to or higher than 0.1 wt% and more preferably equal to or higher than 0.2 wt%. Often, the amount of the at least one coating agent which is comprised in the particles according to the present invention, is equal to or less than 15 wt%, preferably equal to or lower than 10 wt% and more preferably equal to or lower than 7 wt%.

In a preferred embodiment of the present invention the coating agent comprises, or consists of, at least one fatty acid, optionally substituted with a hydroxy group. Suitable fatty acids are linear or branched aliphatic carboxylic acids, preferably having 8 to 22 carbon atoms, more preferably 12 to 18 and in particular 16 to 18 carbon atoms, and the salts thereof. The aliphatic chains of the fatty acids may be saturated or unsaturated. Saturated aliphatic fatty acids are preferred. Preferably, the coating agent comprises stearic acid or its salts. Stearic acid is more preferred. Especially preferred is a hydroxy fatty acid or a salt thereof, wherein hydroxy stearic acid is most preferred. Most preferably, the stearic acid bears the hydroxy group in 12-position ($CH_3-(CH_2)_4-CH_2-CHOH-CH_2-(CH_2)_8-CH_2-$ COOH).

Advantageously, the precipitated calcium carbonate particles which are at least partially present in the form of nanofibers or nanochain like agglomerates are treated, either with the fatty acid or hydroxy fatty acid itselves, or with the alkali metal or ammonium salt thereof. To this end, the hydroxy fatty acid often is used as water-based emulsion comprising sodium hydroxide and/or ammonia. Usually, for ease of handling and to ensure the homogeneity of the ultimate product, aqueous solutions or suspensions of a salt of the fatty acid or hydroxy fatty acid are utilized as the means for effecting the subject surface treatment. When a fatty acid, or its salt, is used as coating agent, the added content of the fatty acid is usually equal to or larger than 0.01 wt%, calculated on the basis of the uncoated particles, preferably equal to or larger than 0.1 wt%, and more preferably equal to or larger than 1 wt%. Further, the added content of the fatty acid or its salt usually is equal to or less than 10 wt%, calculated on the basis of the uncoated particles, preferably equal to or less than 7 wt%, and more preferably equal to or less than 4 wt%. In particular, the preferred content range for hydroxystearin is from 0.1 to 7 wt%, and more preferably from 1 to 4 wt%.

In another preferred embodiment, the precipitated calcium carbonate particles are at least partially coated with at least one sulfur organic compound. Suitable sulfur organic compounds are, for example, an organic sulfonic acid, its ester and/or salt alkylsulfonic acids, alkylsulfosuccinates, alkylsulfates and the salts thereof, their esters and/or salts. In particular, organic sulfonic acids can be alkylsulfonic acids, arylsulfonic acids, alkylarylsulfonic acids, the esters and salts thereof.

For the purpose of the specification, the term "aryl" denotes an aromatic mono- or bicyclic hydrocarbon comprising 6 to 10 carbon atoms, such as phenyl and naphthyl, optionally substituted with at least one halogen atom.

For the purpose of the specification, the term "alkylaryl" denotes an "alkyl" residue as defined above covalently linked to an "aryl"-residue as defined above, such as $-CH_2C_6H_5$, $-CH_2CH_2C_6H_5$, $-CH_2CH_2CH_2C_6H_5$, and the like.

In a preferred embodiment the coating agent comprises a compound represented by general formula (I-A) or (I-B)

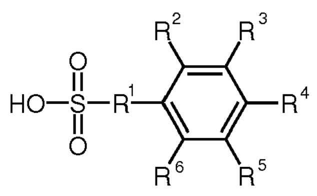

(I-A)

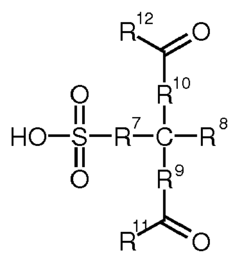

(I-B)

wherein

$R^1$, $R^7$, $R^9$ and $R^{10}$ are independently a single bond, -O-, $-C_1-C_{18}$-alkylene- or-$C_2-C_{18}$-alkenylene- (wherein in the alkylene- or in the alkenylene-chain optionally 1, 2 or 3 $-CH_2-$ groups may be replaced by -O-); $R^2$, $R^3$, $R^4$, $R^5$ and $R^6$ are independently -H, $-C_1-C_{18}$-alkyl (wherein in the alkyl-chain optionally 1, 2 or 3 $-CH_2-$ groups may be replaced by -O-), -OH, -F,-Cl, -CN, $-CO_2H$, $-CO-C_1-C_6$-alkyl, $-CO_2-C_1-C_6$-alkyl, $-O-CO-C_1-C_6$-alkyl, - $NO_2$, $-NH_2$, $-NH-C_1-C_6$-alkyl or $-N(C_1-C_6\text{-alkyl})_2$;

$R^8$ is -H or $-C_1-C_6$-alkyl; and

$R^{11}$ and $R^{12}$ are independently -H, $-C_1-C_{18}$-alkyl (wherein in the alkyl-chain optionally 1, 2 or 3 $-CH_2-$ groups may be

replaced by -O-),$-NH_2$, $-NH-C_1-C_6$-alkyl or $-N(C_1-C_6\text{-alkyl})_2$.

For the purpose of the specification, the term "alkylene" denotes bivalent linear or branched alkylene chains, such as $-CH_2-$, $-CH_2CH_2-$, $-CH_2CH_2CH_2-$, $-CH_2CH_2CH_2CH_2-$, $-CH(CH_3)-$, $-CH_2CH(CH_3)-$, $-CH_2CH_2CH(CH_3)-$, $-CH_2CH(CH_3)CH_2-$, and the like.

For the purpose of the specification, the term "alkenylene" denotes bivalent linear or branched alkenylene chains, such as -CH=CH-, $-CH_2CH=CH-$, $-CH_2CH_2CH=CH-$, $-CH=C(CH_3)-$, $-CH_2CH=C(CH_3)-$, $-CH=C(CH_3)CH_2-$, and the like.

Preferably, in the compound represented by general formula (I-A) or (I-B) $R^1$, $R^7$, $R^9$ and $R^{10}$ are independently a single bond or $-C_1-C_6$-alkylene- (wherein in the alkylene-chain optionally 1 or 2 $-CH_2-$ groups may be replaced by -O-); $R^2$, $R^3$, $R^4$, $R^5$ and $R^6$ are independently -H or $-C_1-C_{18}$-alkyl (wherein in the alkyl-chain optionally 1, 2 or 3 $-CH_2-$ groups may be replaced by -O-); $R^8$ is -H or $-C_1-C_6$-alkyl; and
$R^{11}$ and $R^{12}$ are independently $-C_1-C_{12}$-alkyl (wherein in the alkyl-chain optionally 1, 2 or 3 $-CH_2-$ groups may be replaced by -O-).

More preferably, in the compound represented by general formula (I-A) or (I-B)
$R^1$ and $R^7$ are a single bond;
$R^2$, $R^3$, $R^5$ and $R^6$ are -H
$R^4$ is -H or $-C_1-C_6$-alkyl;
$R^8$ is -H;
$R^9$ and $R^{10}$ are independently a single bond or $-CH_2-$; and
$R^{11}$ and $R^{12}$ are independently $-C_1-C_{12}$-alkoxy.

For the purpose of the specification, the term "alkoxy" denotes an "alkyl" residue as defined above covalently linked to an oxygen atom, such as$-OCH_3$, $-OCH_2CH_3$, and the like.

In a most preferred embodiment, the at least one coating agent is a salt, preferably a sodium salt, of alkylbenzenesulfonic acid (alkylbenzenesulfonate). The preferred alkylbenzenesulfonate, which also corresponds to formula (I-A), is constituted of 12-15 wt% of a toluene sulfonic acid moiety, with R1 being a covalent bond, and one of $R^2$ to $R^6$ being $-CH_3$, and the remaining residues $R^2$ to $R^6$ being optionally branched alkyl. Preferably, the optionally branched alkyl chains are distributed in the following way:

- C9: < 1 wt. %
- C10: 8-16 wt.%
- C11: 26-38 wt.%
- C12: 26-38wt.%
- C13: 15-27wt. %
- C14: < 1 wt. %

When a sulfur organic compound, or its salt, is used as coating agent, the added content of the sulfur organic compound is usually equal to or larger than 0.01 wt%, calculated on the basis of the uncoated particles, preferably equal to or larger than 0.05 wt%, and more preferably equal to or larger than 0.1 wt%. Further, added content of the fatty acid or its salt usually is equal to or less than 10 wt%, calculated on the basis of the uncoated particles, preferably equal to or less than 7 wt%, and more preferably equal to or less than 4 wt%.In particular, the preferred content range for alkylbenzenesulfonic acids or their salts is from 0.01 to 2 wt%, and more preferably from 0.05 to 0.5 wt%.

When a sulfur organic compound, or its salt, is used as coating agent, it is usually dissolved or suspended in an aqueous system. Such an aqueous system can contain, apart from water, other additives, such as salts (for example sodium sulfate and/or sodium chloride), and auxiliary additives such as an oil. Usually the content of such additional additives and auxiliary additives in such a suspension or solution does not exceed 9 wt%.

According to a preferred embodiment, the precipitated calcium carbonate further comprises at least one crystallization controller, wherein the crystallization controller preferably is selected from the group consisting of polyacrylic acid, salts thereof and mixtures thereof, citric acid, sodium dioctylsulfosuccinate, polyaspartic acid and Ethylenediaminetetraacetic acid (EDTA). Polyacrylic acid, salts thereof and mixtures thereof, and Ethylenediaminetetraacetic acid (EDTA) are the most preferred crystallization controllers.

When polyacrylic acid, salts thereof and mixtures thereof are present in the precipitated calcium carbonate particles, generally the molecular weight polyacrylic acid or its salt, in particular sodium salt, is from 500 and 15 000 g/mol. The molecular weight usually is equal to or lager than 500 g/mol, preferably equal to or larger than 700 g/mol and especially preferably equal to or larger than 1000 g/mol. Generally the molecular weight is equal to or less than 15 000 g/mol, preferably equal to or larger than 10 000 g/mol and especially preferably equal to or larger than 5000 g/mol. Molecular weights of from 1000 to 3500 g/mol are particularly preferred. If the polyacrylic acid is present as salt, such as the sodium salt, the degree of acid neutralization by its cation, in particular sodium, can be from 0 to 100 %.

Polymeric compositions, in particular plastisol compositions, comprising the precipitated calcium carbonate

particles which are at least partially present in the form of nanofibers or nanochain like agglomerates which are at least partially coated with at least one coating agent, as filler generally display an increased yield stress, and thus, improved rheological properties. Generally, such compositions display a yield stress value of from 250 to 600 Pa, measured according to the measurement method described below. Generally, the yield stress value of the compositions comprising the coated particles is equal to or higher than 250 Pa, preferably equal to or higher than 255 Pa and most preferably equal to or higher than 260 Pa. Often, the yield stress value of the compositions comprising the coated particles is equal to or lower than 600 Pa, preferably equal to or lower than 580 Pa and most preferably equal to or lower than 550 Pa. A range of from 280 to 500 Pa is particularly preferred.

The Yield Stress Value is determined using a standardized polymer composition. The polymer composition is defined as follows:

Table 1:

| *formulation* | *Wt %* |
|---|---|
| Plasticizer | 35 |
| Polymer | 25 |
| Solvent | 4 |
| Standard Filler (GCC) | 16 |
| Tested PCC coated particles | 16 |
| Other additives | 4 |

For determining the Yield Stress Value of a polymer composition comprising the precipitated calcium carbonate particles according to the invention, the following procedure is applied:

The PCC samples are stored for minimum one night at 50% relative humidity and 23°C. The mixer used for preparation of the polymer composition is a Dispermat (VMA).

All liquids are weighed and mixing is started at 300 rpm. Polymer powder is added gradually under mixing, mixing can be increased if necessary to a maximum of 800 rpm. Other ingredients are added at a speed which can be raised, if necessary, to a maximum of 800 rpm. After finalized addition, the polymer composition is mixed for 10 minutes at 800 rpm. The yield value of the composition is then determined according to the following method, using a rheometer Kinexus Pro (Malvern) with a concentric cylinder C14.
Interval 1: no shear rate (waiting time) for 300s at 23°C
Interval 2: increase of shear rate from 0 to 131 s-1 in 120s (20 pts)
Interval 3: shear rate at 131 s-1 for 180 s (5 pts)
Interval 4: decrease of shear rate from 131 to 0 s-1 in 120 s (20 pts)
The shear stress is plotted vs. shear rate.
The Bingham model is applied on the downward curve (interval 4) from 20 to 131 s-1. It allows to obtain the yield value, the Bingham viscosity and the correlation coefficient.

According to a further embodiment, the precipitated calcium carbonate has an average primary particle size (dp) of from 15 to 100 nm. Generally, the average primary particle size (dp) of the precipitated calcium carbonate before coating is equal to or more than 15 nm, more preferably equal to or more than 20 nm and even more preferably equal to or more than 25 nm. Often, the average primary particle size (dp) of the precipitated calcium carbonate before coating is equal to or lower than 100 nm, more preferably equal to or lower than 80 nm and even more preferably equal to or lower than 65 nm. A range of from 25 to 60 nm is particularly preferred. Generally, the values apply to the particles before and after coating. The average primary particle size (dp) is typically measured by permeability, which is described below:

dp is determined by permeability measured according to a method derived from BS 4359-2. The basis of this method is the measurement of the air permeability of a pellet, which is analogous to the "Blaine" or the "Lea & Nurse method". The calculation of the dp derives from the Carman & Malherbe formula :

$$q \times L = \frac{1.05\varepsilon^2}{(1-\varepsilon)^2} ds^2 + \frac{2.88\varepsilon^2}{1-\varepsilon} ds$$

with

$$\varepsilon = 1 - \frac{W}{A \times L \times D}$$

It can be shown that the mean particle diameter ds which is determined according to the Carman & Malherbe formula is not absolutely independent from the porosity of the pellet. Consequently, a correction was brought considering the reference porosity $\varepsilon = 0.45$ and the dp was calculated according to the formula :

$$dp = ds \times e^{-3.2(\varepsilon - 0,45)}$$

Definitions and unities are as follows :

q = volumetric rate of air flow passed through the PCC pellet ($cm^3/g$),
$\varepsilon$ = porosity,
W = weight of PCC,
L = thickness of the pellet,
D = density of PCC ($g/cm^3$),
A = area of the cross section of the pellet ($cm^2$),
ds = mean particle diameter according to Carman & Malherbe ($\mu m$), and dp = mean particle diameter according to Solvay ($\mu m$).

According to a further embodiment, the precipitated calcium carbonate before coating has and an aggregate median size D50 of from 0.6 $\mu m$ to 4 $\mu m$. Generally, the aggregate median size D50 of the precipitated calcium carbonate before coating is equal to or more than 0.11 $\mu m$, more preferably equal to or more than 0.2 $\mu m$ and even more preferably equal to or more than 0.3 $\mu m$. Often, the aggregate median size D50 of the precipitated calcium carbonate before coating is equal to or lower than 10 $\mu m$, more preferably equal to or lower than 6 $\mu m$ and even more preferably equal to or lower than 4 $\mu m$. A range of from 0.6 to 4 $\mu m$ is particularly preferred. D50 is measured on the basis of French standard ISO 13317-3, "Particle size sedimentation analysis of a powder by variable height gravity in a liquid. Method by X-ray absorption measurement", in which the general method and the apparatus (Sedigraph) are described. Preparation of the sample influencing the results of the measurement, such preparation method is described as follows:

Preparation of the samples for D50 measurement: 2.7 g of precipitated calcium carbonate are introduced into 50 ml of Na-hexametaphosphate (2 g/L) and the solution is treated by magnetical stirring and ultrasound. For the measurements, a Sedigraph 5100® is equipped with an automatic sampler: as Mastertech 51® from Micromeritics is used. The measurement is performed between 0.1 $\mu m$ (starting diameter) and 100 $\mu m$ (ending diameter). The D50 measurement according to sedimentation method is measured on uncoated calcium carbonate particles.

In a further embodiment, the at least partially coated precipitated calcium carbonate has a loss on drying (LOD) value of equal to or lower than 15 g/kg at 105°C. Generally, the LOD value of the at least partially coated calcium carbonate particles is equal to or more than 0.5 g/kg, preferably equal to or more than 1 g/kg and more preferably equal to or more than 2 g/kg. Often, the LOD value of the at least partially coated calcium carbonate particles is equal to or lower than 15 g/kg, preferably equal to or lower than 12 g/kg and more preferably equal to or lower than 10 g/kg. In a preferred aspect, the LOD value of the at least partially coated calcium carbonate particles is from 4 to 10 g/kg. The LOD is determined according to the following procedure:

Into a weighing bottle, which is pre-dried at 105 °C, about 10 g of precipitated calcium carbonate particles are weighed to the nearest 0.1 mg and heated at about 105°C for 3 hours. The bottles are cooled to approximately 22°C in a desiccator, and weighed to the nearest 0.1 mg. Loss on drying is calculated as g/kg = 1000 * (M1-M2)/M1, wherein M1 is the mass of the particles before drying in g, and M2 the mass of the particles after drying in g. The

result is given in g/kg with 1 decimal.

The invention also relates to a process for the preparation of precipitated calcium carbonate particles being at least partially in the form of nanofibres or nanochain like agglomerates, wherein the particles are at least partially coated with at least one coating agent, which comprises a step of contacting precipitated calcium carbonate particles being at least partially in the form of nanofibres or nanochain like agglomerates with at least one coating agent. In particular, the invention relates to a process for the preparation of precipitated calcium carbonate particles being at least partially in the form of nanofibers or nanochain like agglomerates, wherein the particles are at least partially coated with at least one coating agent, which comprises

(a) Precipitating calcium carbonate particles from a suspension comprising calcium hydroxide and water under conditions providing precipitated calcium carbonate particles being at least partially in the form of nanofibers or nanochain like agglomerates, and
(b) Contacting the particles of calcium carbonate obtained from step (a) with the at least one coating agent.

Generally, the suspension comprising calcium hydroxide and water, which is also denoted as milk of lime (MoL), from which the calcium carbonate particles are precipitated is formed by the reaction of CaO with water or an aqueous medium to form a suspension of Ca(OH)2. Usually, the MoL (Ca(OH)2 in aqueous medium) concentration is from 2 to 20 wt%. In a preferred aspect, the MoL concentration is from 10 to 17 wt%.

The content of calcium carbonate particles in the slurry after precipitation often is equal to or more than 2 wt%, preferably equal to or more than 5 wt% and more preferably equal to or more than 10 wt%. Usually, the content of calcium carbonate particles in the slurry after precipitation is equal to or less than 27 wt%, preferably equal to or less than 25wt% and more preferably equal to or less than 22wt%. The calcium carbonate particles are precipitated by carbonation of the MoL with $CO_2$. Carbon dioxide gas having a concentration of carbon dioxide varying from 3 to 100 % could be used with success. However, it is preferable to use carbon dioxide gas for which the concentration is from 10 to 60 % by volume, especially from 10 to 50 % by volume, and in particular, 23 to 40 % by volume, carbon dioxide gas being diluted with air or other inert gas. Some auxiliary additives might also be further added during or after, preferably after, the carbonation step, such as ascorbic or isoascorbic acid, to reduce yellowness of the resulting calcium carbonate particles.

The precipitation of calcium carbonate particles in step (a) usually is started at a temperature of the MoL which is from 10 to 30 °C. Preferably, the temperature of the MoL at the start of the carbonation is from 12-20°C. At the end of carbonation, a temperature of from 15-70°C, in particular of from 55-65°C is maintained. Heating or cooling is applied during the carbonation when necessary to keep the MoL or slurry temperature in the given range.

The slurry of precipitated calcium carbonate obtained by the carbonation often has a final concentration of equal to or more than 3 wt%, preferably equal to or more than 7 wt%, and most preferably of equal to or more than 15 wt%. Generally, the slurry of precipitated calcium carbonate obtained by the carbonation often has a final concentration of equal to or less than 27 wt%, preferably equal to or less than 25 wt%, and most preferably of equal to or less than 22 wt%. A range of from 15 to 22 wt% is most particularly preferred.

Usually, the suspension is stirred during step (a). If required, the suspension is stirred up to 120 minutes after carbonation is stopped to complete precipitation.

In one embodiment of the present invention, the particles are obtained as a suspension in step (a) and are directly contacted with the at least one coating agent without intermediate separation of the particles from the aqueous phase of the suspension. In another embodiment, the particles are separated/recovered from the aqueous phase of the suspension formed in step (a) before being contacted with the at least one coating agent in step (b).

In a preferred embodiment, the suspension of precipitated calcium carbonate obtained by the carbonation of MoL is contacted with the at least one coating agent. Preferably, one coating agent is added in step (b). Often, the at least one coating agent is added to the suspension formed in step (a) dissolved or suspended in an aqueous medium, thus forming an aqueous coating agent composition comprising at least one coating agent. The hydroxystearin coating agent formulation preferably contains also a base, in a preferred aspect a metal hydroxide, preferably sodium hydroxide, or ammonia. In one aspect, the amount of base present is selected such as to fully neutralize the hydroxystearin. In one embodiment, the at least coating agent or coating agent formulation is added to the precipitated calcium carbonate particles, preferably, the precipitated calcium particle suspension obtained in step (a), in step (b) at a defined temperature. The temperature is dependent on the nature of the at least one coating agent, and generally is in the range of from 10 to 95°C. In one embodiment, when the coating agent formulation is a hydroxystearin formulation, the precipitated calcium carbonate suspension often has a temperature of equal to or more than 50 °C, more preferably equal to or more than 60°C, and even more preferably equal to or more than 70°C. Generally, when the coating agent formulation is a hydroxystearin formulation, the precipitated calcium carbonate suspension often has a temperature of equal to or less than 90 °C, more preferably equal to or less than 85°C, and even more preferably equal to or less than 80°C. The most

preferred temperature range at which the hydroxystearin coating formulation is added to the suspension of precipitated calcium carbonate is from 70 to 80°C. In another embodiment, when the coating agent formulation is a metal, in particular sodium, alkylbenzenesulfonate formulation, the precipitated calcium carbonate suspension often has a temperature of equal to or more than 10 °C, more preferably equal to or more than 20°C, and even more preferably equal to or more than 30°C. Generally, when the coating agent formulation is a metal, in particular sodium, alkylbenzenesulfonate formulation, the precipitated calcium carbonate suspension often has a temperature of equal to or less than 70 °C, more preferably equal to or less than 65°C, and even more preferably equal to or less than 60°C. The most preferred temperature range at which the metal, in particular sodium, alkylbenzenesulfonate coating formulation is added to the suspension of precipitated calcium carbonate is from 45 to 55°C. The temperature of the precipitated calcium carbonate suspension is maintained during the addition of the at least one coating agent or coating agent formulation.

After the at least one coating agent is added to the precipitated calcium carbonate suspension in step (b), the mixture is stirred for a time ranging from 5 to 120 minutes, preferably from 5 to 30 minutes. Often, the temperature given for the addition of the at least one coating agent or coating agent formulation is maintained during stirring.

In another embodiment, the precipitated calcium carbonate is separated from the aqueous phase formed in step (a) by appropriate techniques, such as filtering or spinning and optionally dried, for example by heat application. The wet, separated or optionally dried precipitated calcium carbonate particles are then contacted with the at least one coating agent or coating agent formulation, which are described above, by appropriate techniques, such as milling or stirring, wherein stirring is preferred.

In one preferred embodiment, at least one crystallization controller is present in step (a). More preferably, one crystallization controller is present in step (a). The at least one crystallization controller is added to the milk of lime (MoL) before start of the carbonation. Often, the crystallization controller preferably is selected from the group consisting of polyacrylic acid, salts thereof and mixtures thereof, citric acid, sodium dioctylsulfosuccinate, polyaspartic acid and Ethylenediaminetetraacetic acid (EDTA). Polyacrylic acid, salts thereof and mixtures thereof, and Ethylenediaminetetraacetic acid (EDTA) are the most preferred crystallization controllers.

When polyacrylic acid, salts thereof and mixtures thereof are added before carbonation, generally the molecular weight polyacrylic acid or its salt, in particular sodium salt, is from 500 and 15 000 g/mol. The molecular weight usually is equal to or lager than 500 g/mol, preferably equal to or larger than 700 g/mol and especially preferably equal to or larger than 1000 g/mol. Generally the molecular weight is equal to or less than 15 000 g/mol, preferably equal to or larger than 10 000 g/mol and especially preferably equal to or larger than 5000 g/mol. Molecular weights of from 1000 to 3500 g/mol are particularly preferred. If the polyacrylic acid is present as salt, such as the sodium salt, the degree of acid neutralization by its cation, in particular sodium, can be from 0 to 100 %.

Often, the at least one crystallization controller is added to the milk of lime before carbonation of the milk of lime as an aqueous crystallization controller formulation. In another aspect, the crystallization controller is added to the milk of lime neat. The content of the crystallization controller in the milk of lime is often equal to or more than 0.01 wt%, and generally equal to or less than 10 wt%. In one embodiment, when the at least one crystallization controller is polyacrylic acid, either in its free form or fully or partially neutralized with metal ions such as sodium, the content of polyacrylic acid in the milk of lime is equal to or more than 0.01 wt %, preferably equal to or more than 0.05 wt%, and more preferably equal to or more than 0.1 wt%. Generally, the amount of polyacrylic acid in the milk of lime is equal to or less than 5 wt%, preferably equal to or less than 3 wt%, and more preferably equal to or less than 1 wt%. The most preferred range of polyacrylic acid in the milk of lime is from 0.1 to 1 wt%. Often, when the at least one crystallization controller is polyacrylic acid, either in its free form or fully or partially neutralized with metal ions such as sodium, which is added to the milk of lime in an aqueous formulation, the amount of polyacrylic acid in the polyacrylic acid crystallization controller formulation is equal to or more than 40 wt %, preferably equal to or more than 45 wt%, and more preferably equal to or more than 50 wt%. Generally, the amount of polyacrylic acid in the polyacrylic acid formulation is equal to or less than 70 wt%, preferably equal to or less than 65 wt%, and more preferably equal to or less than 60 wt%. The most preferred range of polyacrylic acid in the polyacrylic acid formulation to be added in step (a) is from 53-55 wt%. In another embodiment, when the at least one crystallization controller is EDTA, the content of EDTA in the milk of lime is equal to or more than 0.1 wt %, preferably equal to or more than 0.2 wt%, and more preferably equal to or more than 0.5 wt%. Generally, the amount of polyacrylic acid in the milk of lime is equal to or less than 10 wt%, preferably equal to or less than 5 wt%, and more preferably equal to or less than 2 wt%. The most preferred range of polyacrylic acid in the milk of lime is from 0.5 to 2 wt%.

According to the preferred embodiment, the aqueous suspension of precipitated calcium carbonate particles as obtained in step (a) are contacted with the coating agent without being separated from the aqueous phase of the suspension. Generally, after completion of step (b), the suspension comprising the at least partially coated precipitated calcium carbonate particles is submitted to a step (c) in which the at least partially coated precipitated calcium carbonate particles are separated from the aqueous phase by appropriate techniques, such as filtration or spinning, and optionally one or more washing steps. In a further optional step (d), the wet cake of at least partially coated precipitated calcium carbonate particles is dried according to appropriate drying techniques, for example in an oven or by infrared radiation.

In a further optional step (e), the at least partially coated precipitated calcium carbonate particles, are milled to avoid the presence of big agglomerates with a size exceeding 100 µm. Optional steps (c) to (e) can be combined in either order and number. A succession of steps (c), (d) and (e) is preferred.

The present invention also concerns the use of the at least partially coated precipitated calcium carbonate particles according to the present invention as additive, for example filler, in paints, plastisols, sealants, paper, food products, nutritional products, pharmaceutical products, inks, lacquers and polymers. The use of the at least partially coated precipitated calcium carbonate particles according to the present invention in plastisols is particularly preferred. The plastisols comprising the at least partially coated precipitated calcium carbonate particles according to the present invention exhibit excellent rheological and other advantageous properties.

The present invention also concerns paint, plastisol, sealant, paper, food product, nutritional product, pharmaceutical product, ink, lacquer, polymer and polymer compositions, comprising at least partially coated precipitated calcium carbonate particles according to the present invention. Plastisols comprising the at least partially coated precipitated calcium carbonate particles according to the present invention are particularly preferred.

In a preferred embodiment, the invention concerns a plastisol, which contains

- the coated particles according to the invention;
- at least one polymer, such as polyvinyl chloride (PVC), e.g. Vestolit, Vinolit,
- optionally at least one plasticizer, such as di-octylphtalate (DOP), di-isononylphtalate (DINP), Adipate, Sebacate
- Optionally at least one solvent, such as derivatives of hydrocarbons
- optionally at least one filler, such as $TiO_2$, $SiO_2$, Natural Calcium Carbonate
- optionally at least one promoter, such as Polyamines, Polyamides
- optionally at least one desiccant, such as CaO and
- optionally further additives.

Preferred embodiments of the compositions according to the invention are summarized here below :

Table 2 :

| [wt.-%] | preferably | more preferably | most preferably |
|---|---|---|---|
| coated particles | 1-50 | 5-30 | 15-25 |
| polymer | 5-75 | 15-50 | 25-35 |
| plasticizer | 10-60 | 20-50 | 30-40 |
| Solvent | 0.5-15 | 1-10 | 3-6 |
| filler | 0-30 | 0-20 | 0-16 |
| promoter | 0-20 | 0-10 | 1-6 |
| desiccant | 0-20 | 0-10 | 1-2 |
| further additives | 0-15 | 0-10 | 0-5 |

The following examples further illustrate the invention but are not to be construed as limiting its scope.

*Example 1 : preparation of precipitated calcium carbonate (PCC)*

A milk of lime with a concentration of 15 % wt. $Ca(OH)_2$ and an initial temperature of 17,5°C was enriched with 0.25 wt% polyacrylic acid (about 2500 g/mol). Carbonation of the milk of lime occurred under a 28 % $CO_2$ flow (diluted with air) at a flow rate of 15 $m^3/h$. The final carbonation temperature was 37.8 °C. A sample of the slurry was filtered, dried in an oven and analyzed with SEM. The sample was found to be constituted of nanochain like agglomerates, combined to form microshell like aggregates. Average diameter and average length were respectively 70 nm and 284 nm. These values are based on the measurement of 10 nanofibers.

*Example 2: coating of precipitated calcium carbonate (PCC) with hydroxystearin*

The slurry obtained in experiment 1 is brought to a temperature of 70°C, and a hydroxystearin emulsion in water is added in an amount corresponding to 30 g/kg of hydroxystearin. The mixture is filtered on a planar filter and is dried in an oven for 36h at 95°C, before milling in an Alpine 160 Z at 14 800 rpm.

Loss on drying for this product at 105°C is 3.4 g/kg; the specific surface area is found to be 22 $m^2/g$.
Fig. 1 shows a SEM micrography of the obtained PCC2.

*Example 3: coating of precipitated calcium carbonate (PCC) with sodium alkylbenzenesulfonate*

The slurry obtained in experiment 1 is brought to a temperature of 50°C, and a sodium alkylbenzenesulfonate emulsion in water is added in an amount corresponding to 10 g/kg of sodium alkylbenzenesulfonate. The mixture is filtered on a planar filter and is dried in an oven for 36h at 95°C, before milling in an Alpine 160 Z at 14 800 rpm.
Loss on drying at 105 °C is 7.9 g/kg for this product and specific surface area of 27 $m^2/g$ is found.
Fig. 2 shows a SEM micrography of the obtained PCC3.

*Example 4: application of precipitated calcium carbonate in plastisol*

*formulation,*

The surface treated calcium carbonate particles obtained by experiment 2 (PCC2) and 3 (PCC 3) were measured according to the Yield Stress Value test described above.
The comparative example is Socal ® 312, which is an ultrafine PCC coated with 28 g/kg stearin. It displays an LOD value of 4.5 g/kg and a specific surface area of 22 $m^2/g$. Socal ® 312 has a calcite rhombohedral crystal structure, and a cubic particle shape. The particles are not agglomerated in nanochains or nanofibres. Fig. 3 shows a SEM micrography of Socal ® 312.

| PCC | Yield Stress Value |
|---|---|
| Comparative Example Socal ® 312 | 218 |
| PCC 2 | 285 |
| PCC 3 | 300 |

## Claims

1. Precipitated calcium carbonate particles being at least partially in the form of nanofibers or nanochain like agglomerates, wherein the particles are at least partially coated with at least one coating agent.

2. Precipitated calcium carbonate particles according to claim 1, wherein the nanofibers or nanochain like agglomerates have an average specific surface area of equal to or lower than 60 $m^2/g$, preferably equal to or lower than 50 $m^2/g$ before coating (measured by the BET technique described in ISO 9277).

3. The precipitated calcium carbonate according to claim 1 or 2, wherein the nanofibers or nanochain like agglomerates are further aggregated to form microshells or microsheaves.

4. The precipitated calcium carbonate according to any one of claims 1 to 3, wherein the at least one coating agent is selected from the group consisting of sulfur organic compounds, fatty acids and the salts thereof, preferably wherein the at least one coating agent is a fatty acid or salt thereof or an organic sulfonic acid, its ester and/or salt.

5. The precipitated calcium carbonate according to any one of claims 1 to 4 wherein the at least one coating agent is comprised in an, if applicable combined, amount of from 0.01 wt% to 15 wt%.

6. The precipitated calcium carbonate according to any one of claims 1 to 5 wherein the precipitated calcium carbonate further comprises at least one crystallization controller, wherein the crystallization controller preferably is selected from the group consisting of polyacrylic acid, salts thereof and mixtures thereof, citric acid, sodium dioctylsulfosuccinate, polyaspartic acid and Ethylenediaminetetraacetic acid (EDTA).

7. The precipitated calcium carbonate according to any one of claims 1 to 6 wherein a standard polymer composition comprising the precipitated calcium carbonate displays a yield stress value of from 250 to 600 Pa..

8. The precipitated calcium carbonate according to any one of claims 1 to 7 wherein the precipitated calcium carbonate

before coating has an average primary particle size (dp) of from 15 to 100 nm, and an aggregate median size of from 0.11 μm to 10 μm.

**9.** The precipitated calcium carbonate according to any one of claims 1 to 8 wherein the precipitated calcium carbonate has a loss on drying value of equal to or lower than 15 g/kg at 105°C.

**10.** Process for the preparation of precipitated calcium carbonate particles being at least partially in the form of nanofibres or nanochain like agglomerates, wherein the particles are at least partially coated with at least one coating agent, which comprises a step of contacting precipitated calcium carbonate particles being at least partially in the form of nanofibres or nanochain like agglomerates with at least one coating agent.

**11.** Process according to claim 10, which comprises

(a) Precipitating calcium carbonate particles from a suspension comprising calcium hydroxide and water under conditions providing precipitated calcium carbonate particles being at least partially in the form of nanofibers or nanochain like agglomerates, and
(b) Contacting the particles of calcium carbonate obtained from step (a) with the at least one coating agent.

**12.** Process according to claim 11, wherein the suspension further comprises at least one crystallization controller, wherein the crystallization controller preferably is selected from the group consisting of polyacrylic acid, salts thereof and mixtures thereof, citric acid, sodium dioctylsulfosuccinate, polyaspartic acid and Ethylenediaminetetraacetic acid (EDTA).

**13.** Process according to any one of claims 10 to 12 wherein the least one coating agent is selected from the group consisting of organic sulfonic acids, alkylsulfates, fatty acids and the salts thereof, preferably wherein the at least one coating agent is a fatty acid or salt thereof or an organic sulfonic acid.

**14.** Process according any one of claims 10 to 13 wherein a temperature of from 70 to 80°C, when the at least one coating agent is hydroxystearin, and from 45 to 55°C, when the at least one coating agent is a metal, in particular sodium, alkylbenzenesulfonate, is maintained in step (b).

**15.** Use of precipitated calcium carbonate particles according to any one of claims 1 to 9 as additive, for example filler, in paints, plastisols, sealants, paper, food products, nutritional products, pharmaceutical products, inks, lacquers and polymers, in particular in plastisols.

**16.** Paint, plastisol, sealant, paper, food product, nutritional product, pharmaceutical product, ink, lacquer, polymer and polymer compositions, in particular plastisol, comprising precipitated calcium carbonate particles according to any one of claims 1 to 9.

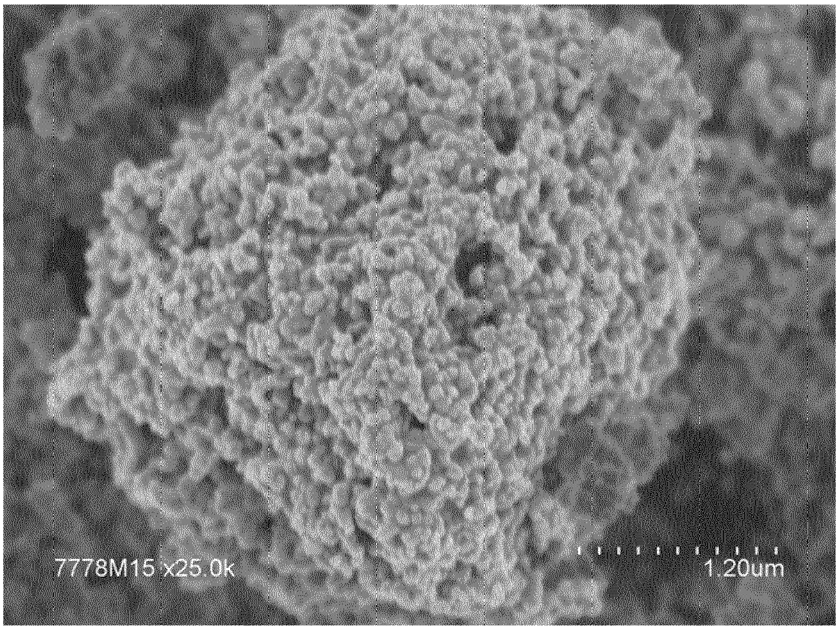


Fig. 1: SEM micrography of PCC2.

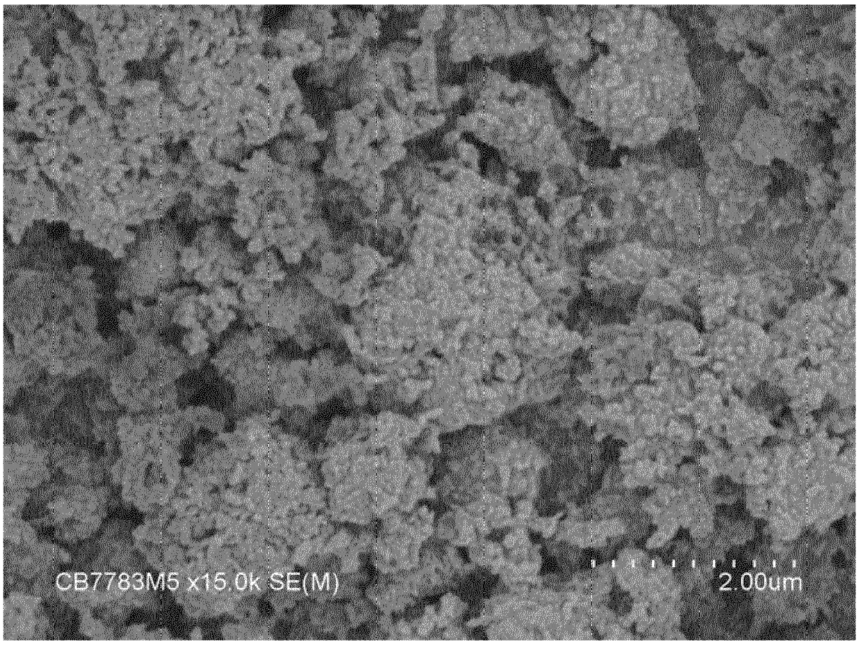


Fig. 2: SEM micrography of PCC3.

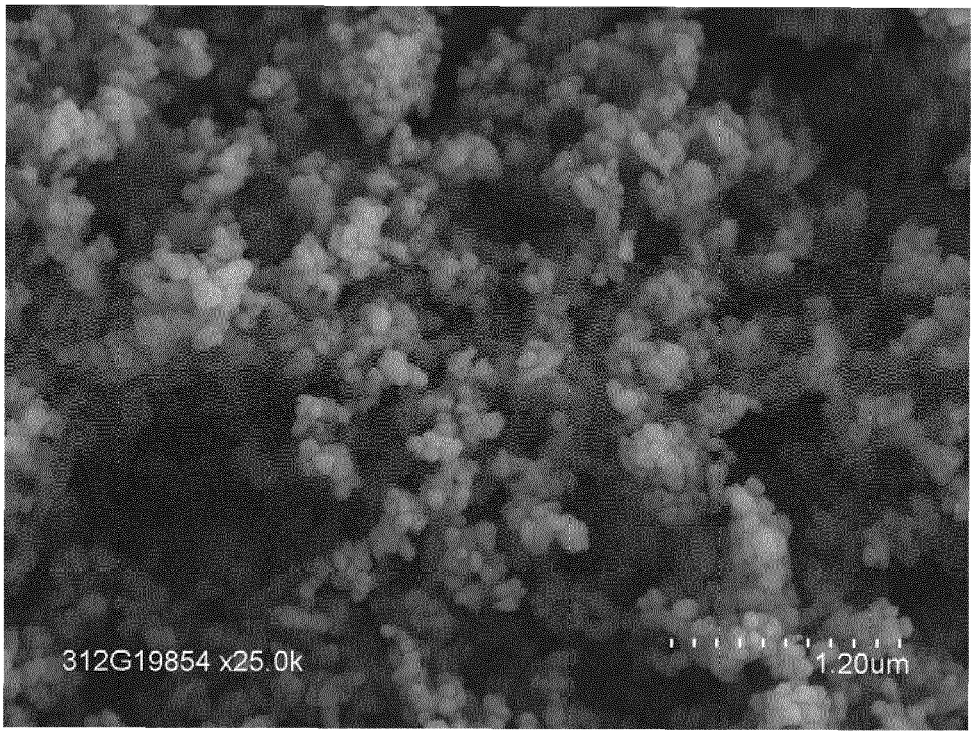


Fig. 3: SEM micrography of Socal ® 312.

Europäisches Patentamt
European Patent Office
Office européen des brevets

# EUROPEAN SEARCH REPORT

Application Number
EP 15 17 9176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/014878 A2 (SOLVAY [BE]; CERVELLATI GIANFRANCO [IT]; ROSA ROBERTO [IT]) 8 February 2007 (2007-02-08) * claims 1,4-7,10,11 * * page 7, line 6 - line 13 * * page 9, line 1 - line 9 * ----- | 1-16 | INV. C09C1/02 C09D7/12 C08K3/26 C08K7/08 |
| A | US 2004/166047 A1 (VOGELS CLAUDE [BE] ET AL) 26 August 2004 (2004-08-26) * examples * * claims * ----- | 1-16 | |
| X | WO 2006/134080 A1 (SOLVAY [BE]; CERVELLATI GIANFRANCO [IT]; ROSA ROBERTO [IT]) 21 December 2006 (2006-12-21) * page 4, line 24 - page 5, line 30 * * claims 1-6,8,16-22 * ----- | 1-16 | |
| X | US 2010/041809 A1 (CAVALIER KARINE [FR] ET AL) 18 February 2010 (2010-02-18) * paragraph [0025] * * paragraph [0026] - paragraph [0029] * * paragraph [0040] * * paragraph [0074] - paragraph [0079] * * examples 1-6 * * claims * ----- | 1-16 | TECHNICAL FIELDS SEARCHED (IPC): C08K C09C C09D |

The present search report has been drawn up for all claims

1

| Place of search | Date of completion of the search | Examiner |
|---|---|---|
| Munich | 15 December 2015 | Russell, Graham |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

## ANNEX TO THE EUROPEAN SEARCH REPORT ON EUROPEAN PATENT APPLICATION NO. EP 15 17 9176

This annex lists the patent family members relating to the patent documents cited in the above-mentioned European search report. The members are as contained in the European Patent Office EDP file on The European Patent Office is in no way liable for these particulars which are merely given for the purpose of information.

15-12-2015

| Patent document cited in search report | Publication date | Patent family member(s) | Publication date |
|---|---|---|---|
| WO 2007014878 A2 | 08-02-2007 | EP 1913100 A2 | 23-04-2008 |
| | | WO 2007014878 A2 | 08-02-2007 |
| US 2004166047 A1 | 26-08-2004 | CN 1551855 A | 01-12-2004 |
| | | EP 1406838 A1 | 14-04-2004 |
| | | ES 2553453 T3 | 09-12-2015 |
| | | FR 2826950 A1 | 10-01-2003 |
| | | JP 4515759 B2 | 04-08-2010 |
| | | JP 2004533396 A | 04-11-2004 |
| | | KR 20040030727 A | 09-04-2004 |
| | | US 2004166047 A1 | 26-08-2004 |
| | | US 2009082508 A1 | 26-03-2009 |
| | | WO 03004414 A1 | 16-01-2003 |
| WO 2006134080 A1 | 21-12-2006 | AR 055061 A1 | 01-08-2007 |
| | | EP 1893546 A1 | 05-03-2008 |
| | | WO 2006134080 A1 | 21-12-2006 |
| US 2010041809 A1 | 18-02-2010 | JP 2008524110 A | 10-07-2008 |
| | | KR 20070099571 A | 09-10-2007 |
| | | KR 20130036350 A | 11-04-2013 |
| | | KR 20150113210 A | 07-10-2015 |
| | | MY 150665 A | 28-02-2014 |
| | | TW I394715 B | 01-05-2013 |
| | | US 2010041809 A1 | 18-02-2010 |
| | | WO 2006067144 A1 | 29-06-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013050495 A
- WO 2006051087 A